# EUROPEAN PATENT APPLICATION

(11) **EP 4 848 294 A1**
(43) Date of publication of application: **02.12.2026**
(21) Application number: 26181715.9
(22) Date of filing: 28.05.2026
(51) Int. Cl.: G16H 10/60, G16H 15/00, G16H 30/40

(54) **MEDICAL DATA ASSOCIATION SYSTEM USING IMAGING DEVICES**

(30) Priority: 28.05.2025 US 202563813014 P
(71) Applicant: Hill-Rom Services, Inc., Batesville, IN 47006 (US)
(72) Inventor: HOCHWORTER, Ryan T., Batesville, 47006 (US); PATEL, Donny, Batesville, 47006 (US); BENASSI, Christopher J., Batesville, 47006 (US)
(74) Representative: Reddie & Grose LLP

(57) **Abstract**

A medical data association system includes a plurality of identifiers, an imaging device, and a controller. The plurality of identifiers includes a first identifier associated with a first medical device and a second identifier different than the first identifier associated with a second medical device. The imaging device is located in a room and configured to detect the plurality of identifiers located within the room. The controller is configured to, upon determining that the first medical device is about to be used for a patient and/or is being used for the patient in the room, associate the first medical device and the patient with one another so that medical data from the first medical device can be populated in an electronic medical record of the patient.

## Description

The present disclosure relates to medical data association systems. More particularly, the present disclosure relates to medical data association systems using imaging devices.

Generally, for medical devices and medical data to be associated with a patient, the medical devices and the medical data need to be manually linked to the patient. For example, a caregiver may scan a barcode on the medical devices and a barcode on a wristband of the patient. In doing so, the medical devices, the medical data therefrom, and the patient are linked. Such an arrangement requires the caregiver to actively associate the medical devices with the patient, which is labor intensive. Further, caregivers may forget to actively associate the medical devices with the patient. Thus, providing an automatic, passive medical data association system would be beneficial.

An apparatus, system, or method may comprise one or more of the following features alone or in any combination:

According to the present disclosure, a medical data association system may be provided. The medical data association system may include a plurality of identifiers, an imaging device, and a controller. The plurality of identifiers may include a first identifier associated with a first medical device and a second identifier different than the first identifier associated with a second medical device. The imaging device may be located in a room and configured to detect the plurality of identifiers located within the room. The first identifier and the second identifier may be visible to the imaging device and substantially imperceptible to unaided human observation. The controller may be in communication with the imaging device and the first medical device. The controller may be configured to, upon determining that the first medical device is about to be used for the patient and/or is being used for the patient, associate the first medical device and the patient with one another so that medical data from the first medical device can be populated in an electronic medical record (EMR) of the patient when transmitted to an EMR computer.

Optionally, the controller may be configured to determine that the first medical device is about to be used and/or is being used based on detection of the first identifier within the room. The controller may be configured to associate the first medical device and the patient with one another without manual association by a caregiver. Upon determining that the first medical device is about to be used and/or is being used, the first medical device may be configured to transmit the medical data to the electronic medical record. The controller may be configured to associate the second medical device with the patient upon detection of the second identifier within the room.

Further optionally, the controller may be configured to associate the second medical device and the patient with one another without manual association by a caregiver. The controller may be configured to compare characteristics of the second medical device with patient characteristics. The controller may be configured to issue an alert in response to the characteristics of the second medical device not aligning with the patient characteristics. The second medical device may be a bed.

If desired, the controller may be configured to disassociate the first medical device and the patient in response to a lack of detection of the first identifier in the room. The controller may be configured to disassociate the second medical device and the patient in response to a lack of detection of the second identifier in the room. The first identifier and the second identifier may be formed of ultraviolet-reactive ink. The first identifier may be applied to the first medical device and the second identifier may be applied to the second medical device.

Alternatively, the imaging device may include an ultraviolet camera. The controller may include a processor and a memory having instructions stored therein which, when executed by the processor, cause the processor to associate the first medical device and the patient with one another. The controller may be configured to receive a first signal from the imaging device indicative of detection of the first identifier within the room. The controller may be configured to receive a second signal from the imaging device indicative of detection of the second identifier within the room. In response to the first signal, the controller may be configured to associate the first medical device and the patient located within the room with one another, and in response to the second signal, the controller may be configured to associate the second medical device and the patient located within the room with one another.

Additionally, in response to the second signal, the controller may be configured to compare characteristics of the second medical device with patient characteristics. The plurality of identifiers may further include a third identifier associated with a caregiver. The controller may be configured to associate the caregiver and the patient with one another in response to detection of the third identifier by the imaging device.

Optionally, the controller may be configured to determine that the first medical device is about to be used and/or is being used based on detection of the first identifier and the third identifier within the room at the same time. The first and second identifiers may be formed of ultraviolet-reactive ink, and the third identifier may include facial features of the caregiver. The first and second identifiers may be formed of ultraviolet-reactive ink, and the third identifier may include an identification badge of the caregiver. The first identifier, the second identifier, and the third identifier may be formed of ultraviolet-reactive ink.

Further according to the present disclosure, a method may be provided. The method may include positioning an imaging device within a room. The method may include detecting a first identifier associated with a first medical device by the imaging device upon entry of the first medical device into the room. The method may include detecting a second identifier associated with a second medical device by the imaging device upon entry of the second medical device into the room. The first identifier and the second identifier may be visible to the imaging device and substantially imperceptible to unaided human observation. The method may include determining, with a controller in communication with the imaging device, that the first medical device is about to be used for and/or is being used for a patient located within the room. The method may include, in response to determining that the first medical device is about to be used and/or is being used, associating, with the controller, the first medical device and the patient with one another. The method may include automatically populating medical data from the first medical device into an electronic medical record (EMR) of the patient.

Optionally, determining that the first medical device is about to be used and/or is being used may include detecting the first identifier within the room. The method may include, after associating the first medical device and the patient with one another, transmitting the medical data from the first medical device to the electronic medical record. The method may include, in response to detecting the second identifier, associating, with the controller, the second medical device and the patient with one another.

Further optionally, the method may include comparing, with the controller, characteristics of the second medical device with patient characteristics. The method may include determining, with the controller, that the characteristics of the second medical device do not align with the patient characteristics. The method may include issuing an alert, with the controller, in response to determining that the characteristics of the second medical device do not align with the patient characteristics. The second medical device may be a bed.

If desired, the method may include disassociating, with the controller, the first medical device and the patient in response to a lack of detection of the first identifier in the room. The method may include disassociating, with the controller, the second medical device and the patient in response to a lack of detection of the second identifier in the room. The method may include applying the first identifier to the first medical device and applying the second identifier to the second medical device via ultraviolet-reactive ink. The imaging device may include an ultraviolet camera.

Alternatively, the method may include detecting a third identifier associated with a caregiver by the imaging device upon entry of the caregiver into the room. The method may include, in response to detecting the third identifier, associating, with the controller, the caregiver and the patient with one another. Determining that the first medical device is about to be used and/or is being used may include detecting the first identifier and the third identifier within the room at the same time.

Additionally, the first and second identifiers may be formed of ultraviolet-reactive ink, and the third identifier may include facial features of the caregiver. The first and second identifiers may be formed of ultraviolet-reactive ink, and the third identifier may include an identification badge of the caregiver. The first identifier, the second identifier, and the third identifier may be formed of ultraviolet-reactive ink.

Further according to the present disclosure, a controller is provided. The controller may include a processor and a memory. The memory may have instructions stored therein executable by the processor to cause the processor to (a) determine that a first identifier associated with a first medical device is located within a room, (b) determine that a second identifier associated with a second medical device is located within the room, (c) determine that the first medical device is about to be used for and/or is being used for a patient within the room, and (d) in response to determining that the first medical device is about to be used for and/or is being used for the patient within the room, associate the first medical device and the patient with one another so that medical data from the first medical device is automatically populated in an electronic medical record of the patient.

Optionally, the instructions stored in the memory may further include instructions executable by the processor to cause the processor to output a signal to the first medical device to cause the first medical device to transmit the medical data to the electronic medical record. The instructions stored in the memory may further include instructions executable by the processor to cause the processor to associate the second medical device and the patient with one another upon determining that the second identifier is located within the room.

Further optionally, the instructions stored in the memory may further include instructions executable by the processor to cause the processor to compare characteristics of the second medical device with patient characteristics. The instructions stored in the memory may further include instructions executable by the processor to cause the processor to issue an alert in response to the characteristics of the second medical device not aligning with the patient characteristics. The instructions stored in the memory may further include instructions executable by the processor to cause the processor to disassociate the first medical device and the patient in response to a lack of detection of the first identifier in the room.

If desired, the instructions stored in the memory may further include instructions executable by the processor to cause the processor to disassociate the second medical device and the patient in response to a lack of detection of the second identifier in the room. The instructions stored in the memory may further include instructions executable by the processor to cause the processor to determine that a third identifier associated with a caregiver is located within the room. The instructions stored in the memory may further include instructions executable by the processor to cause the processor to associate the caregiver and the patient with one another upon determination that the third identifier is located within the room.

The invention will now be further described with reference to the accompanying drawings in which:
FIG. 1 is a diagrammatic view of a medical data association system having a plurality of identifiers associated with medical devices and caregivers and at least one imaging device located in a room that detects the plurality of identifiers;
FIG. 2 is a block diagram of a control system of the medical data association system of FIG. 1 showing a controller in communication with the at least one imaging device, the medical devices, an electronic medical record (EMR) computer, and a hospital database, the controller configured to associate the medical devices and the caregivers to a patient located within the room upon detection of the plurality of identifiers;
FIG. 3 is a block diagram showing inputs to the controller of the medical data association system; and
FIG. 4 is a flow chart showing a process of using the medical data association system of FIG. 1.

The present disclosure relates to a medical data association system 10 that provides automatic and passive association between medical devices 18, 20, medical data, and a patient 24. The medical data association system 10 automatically links the medical devices 18, 20 and the medical data to the patient 24 while the medical devices 18, 20 and the patient 24 are within the same room 32.

Generally, medical devices (and the medical data therefrom) are actively associated with a patient via barcodes and scanners used by a caregiver. For example, the caregiver will scan a barcode on the medical devices and a barcode on a patient wristband to associate the medical devices to the patient. This active association is labor intensive and subject to failure as the caregiver may forget to use the scanner. Thus, the medical data association system 10 automatically associates the medical devices 18, 20 with the patient 24. In this way, active participation of the caregiver 22 in the association process is not required.

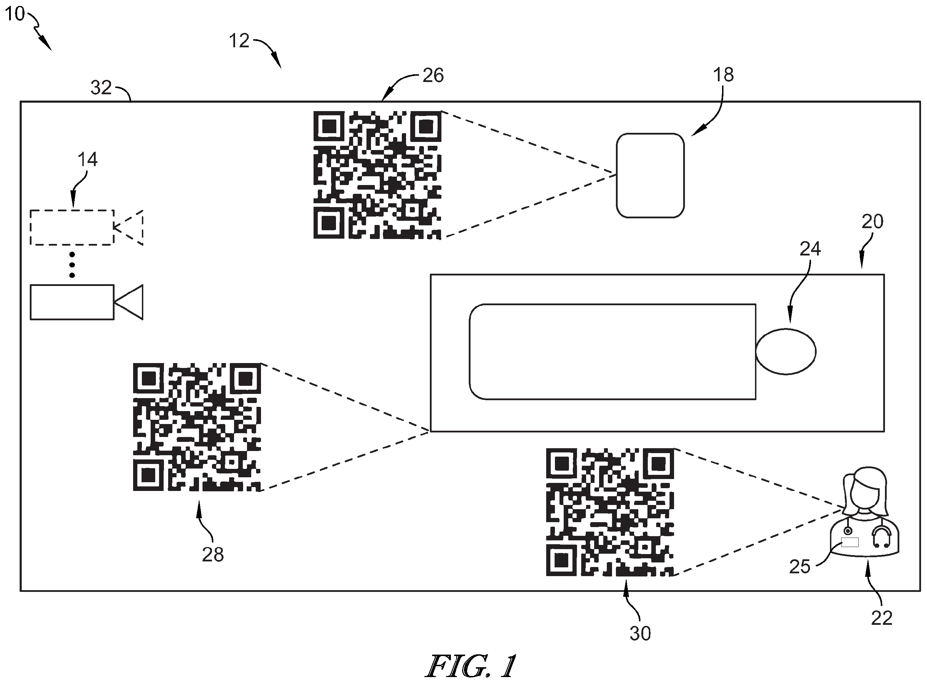

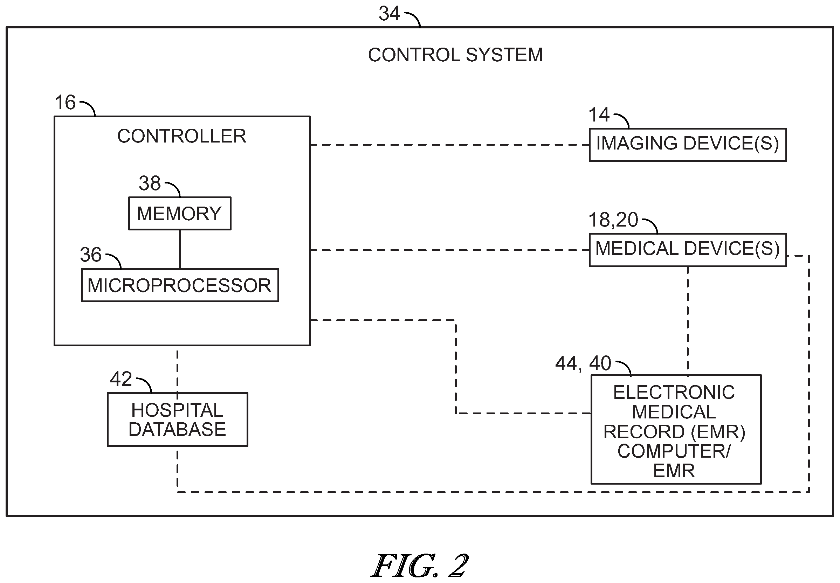

The medical data association system 10 includes a plurality of identifiers 12, at least one imaging device 14, and a controller 16, as shown in FIGS. 1 and 2. Each of the plurality of identifiers 12 is associated with the medical device 18, 20, the caregiver 22, and/or the patient 24. The at least one imaging device 14 is configured to detect the plurality of identifiers 12. The controller 16 is in communication with the imaging device 14.

The plurality of identifiers 12 may include ink, paint, barcodes, quick-read (QR) codes, alphanumeric strings, facial features, badges, or other visual identifiers. In some embodiments, the plurality of identifiers 12 includes a first identifier 26, a second identifier 28, and/or a third identifier 30, as shown in FIG. 1. The first identifier 26 is associated with the first medical device 18. The second identifier 28 is associated with the second medical device 20. The third identifier 30 is associated with the caregiver 22. The identifiers 26, 28, 30 are different from each other so that the identifiers 26, 28, 30 identify different components (i.e., the medical devices 18, 20 and the caregiver 22).

The first identifier 26 is applied to the first medical device 18. For example, the first identifier 26 may be printed onto or adhered to the first medical device 18. The second identifier 28 is applied to the second medical device 20. For example, the second identifier 28 may be printed onto or adhered to the second medical device 20. In some embodiments, the third identifier 30 is applied to the caregiver 22. For example, the third identifier 30 may be printed onto or adhered to the clothing of the caregiver 22 or to an identification badge 25 worn by the caregiver 22. The identifiers 26, 28, 30 may be printed directly onto the first medical device 18, the second medical device 20, the clothing of the caregiver 22, and/or the identification badge 25. The identifiers 26, 28, 30 may be printed directly onto a label that is adhered to, fixed, to, coupled to, and/or attached to the first medical device 18, the second medical device 20, the clothing of the caregiver 22, and/or the identification badge 25.

In some embodiments, the plurality of identifiers 12 is formed of ultraviolet-reactive ink or paint. The ultraviolet-reactive ink or paint may be Camera Flash Ink (CFI) or Camera Flash Pigment (CFP). In some embodiments, such as embodiments using ultraviolet-reactive ink or paint, the plurality of identifiers 12 is visible to the imaging device 14 and generally not interpretable by a human eye. Generally not interpretable by a human eye may mean that the plurality of identifiers 12 is invisible to the human eye. Generally not interpretable by a human eye may mean that the plurality of identifiers 12 is generally not visible to a human eye. In other words, the plurality of identifiers 12 is substantially outside a range of human visible perception such that the plurality of identifiers 12 is not observable by a human observer with unaided vision under standard conditions. In this way, the human may, in particular circumstances (i.e., lighting, viewing angle, etc.), be able to discern some perceptible discoloration on the medical devices 18, 20 indicative of the plurality of identifiers 12. Put another way, the plurality of identifiers 12 is substantially imperceptible to unaided human observation - referring to markings that are not detectable by a human observer possessing ordinary visual acuity under ambient visible-light conditions without specialized illumination, optical filtering, magnification, computational processing, and/or imaging equipment.

In some embodiments, the plurality of identifiers 12 is formed of infrared ink or paint. In some embodiments, such as embodiments using infrared ink or paint, the plurality of identifiers 12 is visible to the imaging device 14 and generally not interpretable by a human eye. Generally not interpretable by a human eye may mean that the plurality of identifiers 12 is invisible to a human eye. Generally not interpretable by a human eye may mean that the plurality of identifiers 12 is generally not visible to a human eye. In other words, the plurality of identifiers 12 is substantially outside a range of human visible perception such that the plurality of identifiers 12 is not observable by a human observer with unaided vision under standard conditions. In this way, the human may, in particular circumstances (i.e., lighting, viewing angle, etc.), be able to discern some perceptible discoloration on the medical devices 18, 20 indicative of the plurality of identifiers 12. Put another way, the plurality of identifiers 12 is substantially imperceptible to unaided human observation - referring to markings that are not detectable by a human observer possessing ordinary visual acuity under ambient visible-light conditions without specialized illumination, optical filtering, magnification, computational processing, and/or imaging equipment.

In some embodiments, the plurality of identifiers 12 includes a marker that forms a contrast pattern with the substrate on which it is coupled in a wavelength of light that is substantially outside the range of human visible perception. In this way, the at least one imaging device 14, such as an ultraviolet sensitive imager, an infrared sensitive imager, a short-wave infrared imager, a near infrared imager, a middle infrared imager, a far infrared imager, etc., is configured to detect the plurality of identifiers 12.

In some embodiments, the plurality of identifiers 12 includes a marker that forms a pattern with the substrate on which it is coupled in visible wavelengths of light when exposed to higher than ambient levels of non-visible light. In this way, the at least one imaging device 14, such as an ultraviolet sensitive imager, is configured to detect the plurality of identifiers 12.

In some embodiments, the plurality of identifiers 12 includes a marker that is embedded within an image. In this way, the plurality of identifiers 12 is hidden within the image and machine detectable (i.e., steganographic encodement). The marker is technically visible, but it is indiscernible and/or imperceptible from the image without electronic assistance. In this way, the at least one imaging device 14, such as an imager and computational processing software, is configured to detect the plurality of identifiers 12.

In some embodiments, the plurality of identifiers 12 is formed of visible indicia. The visible indicia may include visible ink, grooves, recesses, patterns, markers, or any other visible indicia. In some embodiments, such as embodiments using visible indicia, the plurality of identifiers 12 is visible to the imaging device 14 and visible to a human eye.

In some embodiments, the first and second identifiers 26, 28 are formed of ultraviolet-reactive ink, ultraviolet-reactive paint, or visual indicia and the third identifier 30 comprises facial features of the caregiver 22. In this way, the caregiver 22 is identified via facial recognition.

In some embodiments, the first and second identifiers 26, 28 are formed of ultraviolet-reactive ink, ultraviolet-reactive paint, or visual indicia and the third identifier 30 comprises an identification badge 25 of the caregiver 22. In such an embodiment, the identification badge 25 of the caregiver 22 may be tracked through a real-time location system (RTLS) via wireless signals emitted from the identification badge 25. Identification badges 25 of the RTLS are sometimes referred to synonymously as tags.

In some embodiments, the first, second, and third identifiers 26, 28, 30 are formed of ultraviolet-reactive ink, ultraviolent-reactive paint, or visual indicia. In such an embodiment, the third identifier 30 may be formed on or adhered to the clothing of the caregiver 22 or formed on or adhered to the identification badge 25 worn by the caregiver 22. For example, the ultraviolet-reactive ink, the ultraviolent-reactive paint, or the visual indicia may be printed on to or adhered to the identification badge 25. In such an embodiment, the identification badge 25 of the caregiver 22 may also be tracked through the RTLS via the wireless signals emitted from the identification badge 25 and received by receivers of the RTLS located throughout a facility. In this way, the caregiver 22 may be tracked via the RTLS and via the third identifier 30. The identification badge 25, thus, provides for dual tracking of the caregiver 22.

In some embodiments, the third identifier 30 comprises facial features of the caregiver 22 and ultraviolet-reactive ink, ultraviolent-reactive paint, or visual indicia. In this way, the caregiver 22 may be tracked in multiple ways. For example, the caregiver 22 may be identified, detected, and tracked via the facial features, via the RTLS, and/or via the ultraviolet-reactive in, ultraviolent-reactive paint, or visual indicia. The multi-way detection and tracking provides for back-up detection and tracking methods in the event one of the methods is not working or malfunctioning.

Though shown and described with three identifiers 12, the plurality of identifiers 12 may include any number of identifiers associated with medical devices and caregivers. In some embodiments, the plurality of identifiers 12 includes an identifier (not shown) associated with the patient 24. For example, the clothing of the patient 24 may be formed to include the identifier or the identifier may be adhered to the clothing of the patient 24. As another example, the identifier may comprise facial features of the patient 24.

The at least one imaging device 14 is located in a room 32 at the facility, as shown in FIG. 1. For example, the at least one imaging device 14 may be fixed to a wall or a ceiling within the room 32 such that the at least one imaging device 14 is not moved around by the caregiver 22 or another user. In some embodiments, the imaging device 14 is coupled to the wall or the ceiling and free to move relative to its mounting location under its own control to scan the room 32. In some embodiments, the at least one imaging device 14 is not located in the room 32. For example, the at least one imaging device 14 may be located in the hallway, within the walls of the room 32, or in an adjacent room. In this way, the at least one imaging device 14 may detect the plurality of identifiers 12 through walls. The at least one imaging device 14 may be embedded in the wall of the room 32 such that the at least one imaging device 14 is substantially hidden from the patient 24 in the room 32. For example, the wall may have a translucent portion that the at least one imaging device 14 may detect the plurality of identifiers 12 through. As another example, the wall may be opaque, and the at least one imaging device 14 may detect the plurality of identifiers 12 through the opaque wall.

The facility may be a hospital, a rehabilitation center, a nursing home, a post-surgical recovery center, an urgent care, a doctor's office, etc. The at least one imaging device 14 is configured to detect the plurality of identifiers 12 located within the room 32. For example, the medical devices 18, 20 and the caregiver 22 may move around the facility between different rooms, and the at least one imaging device 14 may detect the medical devices 18, 20 and the caregiver 22 as the medical devices 18, 20 and the caregiver 22 enter or exit the room 32.

In some embodiments, the at least one imaging device 14 includes an ultraviolet camera that can detect ultraviolet-reactive ink or ultraviolet-reactive paint. In such an embodiment, the at least one imaging device 14 illuminates the plurality of identifiers 12 with a camera flash. The illumination of the plurality of identifiers 12 allows the at least one imaging device 14 to capture each of the plurality of identifiers 12.

In some embodiments, the at least one imaging device 14 includes a camera that can detect ultraviolet-reactive ink, ultraviolet-reactive paint, visible indicia, and/or facial features. In some embodiments, the at least one imaging device 14 includes an infrared camera that can detect infrared ink and/or thermal images. In some embodiments, the at least one imaging device 14 includes a camera that can detect ultraviolet-reactive ink, ultraviolet-reactive paint, visible indicia, infrared ink, thermal images, and/or facial features.

Though shown and described with one imaging device 14, the at least one imaging device 14 may include any number of imaging devices fixed within the room 32. In this way, the room 32 may include different imaging devices 14 focused on different areas of the room 32. The different imaging devices 14 may be configured to capture identifiers 12 in the different areas of the room 32. For example, one imaging device 14 may be pointed toward a door leading into the room 32, another imaging device 14 may be pointed toward a center of the room 32, and yet another imaging device 14 may be pointed downwardly from the ceiling of the room 32, to name a few options.

As shown in FIG. 2, the medical data association system 10 includes the controller 16 as part of a control system 34. The controller 16 includes a microprocessor 36 and a memory 38. The controller 16 is in communication with the at least one imaging device 14 and the medical devices 18, 20.

The controller 16 is configured to receive signals from the at least one imaging device 14 in response to the imaging device 14 sensing, identifying, or detecting the plurality of identifiers 12 in the room 32. For example, if the caregiver 22 enters the room 32, the third identifier 30 may be detected by the at least one imaging device 14. As another example, if the first medical device 18 enters the room 32, the first identifier 26 may be detected by the at least one imaging device 14. As another example, if the second medical device 20 is already located within the room 32, the at least one imaging device 14 may detect the second identifier 28. The at least one imaging device 14 then communicates with the controller 16 regarding the detection of the plurality of identifiers 12.

The controller 16 is also in communication with a hospital database 42, as shown in FIG. 2. From the hospital database 42, the controller 16 receives information regarding the room 32, the at least one imaging device 14, and the patient 24. For example, the controller 16 receives a room number of the room 32 (i.e., room #48), an identification of the at least one imaging device 14 located within that room 32, and an identification of the patient 24 located within the room 32. Because the patient 24 is linked to the room 32 via the hospital database 42, the patient 24 may not need to be associated with one of the plurality of identifiers 12. The hospital database 42 includes separate databases, such as in different computers or servers, in some embodiments.

The identification of the patient 24 may include a name of the patient 24, an identifying number of the patient 24, and/or patient characteristics. The patient characteristics may include age, allergies, medical history, injuries, illnesses, prescribed therapies, treatments, etc.

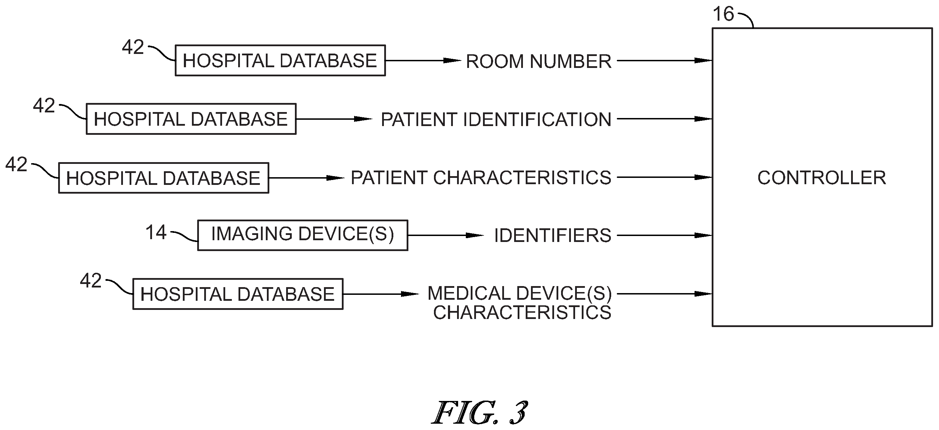

The memory 38 of the controller 16 stores data therein regarding the room 32 and the at least one imaging device 14 in the room 32, as suggested in FIG. 3. As the at least one imaging device 14 communicates with the controller 16 regarding detection of the plurality of identifiers 12, the memory 38 of the controller 16 stores and links the plurality of identifiers 12 to the room 32.

The memory 38 of the controller 16 stores information therein regarding the plurality of identifiers 12 and the associated medical devices 18, 20 and caregiver 22. For example, the memory 38 has the first identifier 26 and the associated first medical device 18 stored therein. In this way, if the first identifier 26 is detected by the imaging device 14 and communicated to the controller 16, the controller 16 determines that, because the first identifier 26 was detected, the first medical device 18 is located within the room 32.

From the hospital database 42, the controller 16 receives information regarding the medical devices 18, 20 or the caregiver 22. The information may include medical device characteristics and/or caregiver characteristics. For example, the controller 16 receives medical device characteristics of the first medical device 18 when the first medical device 18 is detected, as shown in FIG. 3. The medical device characteristics are associated with the specific first medical device 18 such that the characteristics may be different for each medical device 18, 20.

The controller 16, thus, receives, determines, and/or stores the caregiver 22 identification, the medical device 18, 20 identification, the patient 24 identification, and the room 32, as suggested in FIG. 3. The controller 16 links and associates this information.

The controller 16 is configured to determine that one or more of the medical devices 18, 20 is about to be used for the patient 24 and/or is being used for the patient 24. For example, the controller 16 may determine that the first medical device 18 is about to be used for or is being used for the patient 24 upon detection of the first identifier 26. In this way, if the first medical device 18 enters the room 32 (or is already in the room 32) and the imaging device 14 detects the first identifier 26, the controller 16 determines that the first medical device 18 is about to be used for the patient 24.

As another example, the controller 16 may determine that the first medical device 18 is about to be used for and/or is being used for the patient 24 upon detection of the first identifier 26 and detection of the third identifier 30. In this way, if the first medical device 18 and the caregiver 22 are both located within the room 32 and the imaging device 14 detects the first identifier 26 and the third identifier 30, the controller 16 determines that the first medical device 18 is about to be used.

As another example, the controller 16 may determine that the first medical device 18 is about to be used for and/or is being used for the patient 24 upon detection of a threshold proximity between the first medical device 18 and the patient 24. In this way, if the first medical device 18 is located within the threshold proximity to the patient 24 in the room 32, the controller 16 determines that the first medical device 18 is about to be used for and/or is being used for the patient 24.

As another example, the controller 16 may determine that the first medical device 18 is about to be used for and/or is being used for the patient 24 upon detection of a threshold proximity between the first medical device 18 and the caregiver 22. In this way, if the caregiver 22 is located within the threshold proximity to the first medical device 18 in the room 32, the controller 16 determines that the first medical device 18 is about to be used for and/or is being used for the patient 24.

The threshold proximity between the first medical device 18 and the patient 24 or the caregiver 22 and the first medical device 18 may be determined using any known method. For example, the controller 16 may determine an approximate distance between the first medical device 18 and the patient 24 or the caregiver 22 and the first medical device 18 based on captured images from the at least one imaging device 14. As another example, a first imaging device 14 may detect the plurality of identifiers 12 and a separate imaging device may be mounted to each of the medical devices 18, 20. The separate imaging device may be used to approximate the distance between the first medical device 18 and the patient 24 or the caregiver 22 and the first medical device 18.

Upon determining that the first medical device 18 is about to be used and/or is being used, the controller 16 associates the first medical device 18 and the patient 24 with one another so that medical data from the first medical device 18 can be or is automatically populated in an electronic medical record 40 of the patient 24 when transmitted to an EMR computer or server 44. In some embodiments, the controller 16 receives the medical data from the first medical device 18 and transmits the medical data to the EMR computer or server 44 for population in the EMR 40. The controller 16 may also transmit the patient identification to the EMR computer or server 44 before the medical data is transmitted thereto or simultaneously with the medical data so that the medical data may be properly populated in the EMR 40. In some embodiments, the first medical device 18 transmits the medical data to the EMR computer or server 44 for population in the EMR 40. The controller 16 may also transmit the patient identification to the EMR computer or server 44 before the medical data is transmitted thereto by the first medical device 18 or simultaneously with the medical data so that the medical data may be properly populated in the EMR 40.

The controller 16 may also associate the second medical device 20 and the patient 24 with one another so that medical data from the second medical device 20 can be or is automatically populated in the electronic medical record 40 of the patient 24 when transmitted to the EMR computer or server 44. In some embodiments, the controller 16 receives the medical data from the second medical device 20 and transmits the medical data to the EMR computer or server 44 for population in the EMR 40. The controller 16 may also transmit the patient identification to the EMR computer or server 44 before the medical data is transmitted thereto or simultaneously with the medical data so that the medical data may be properly populated in the EMR 40. In some embodiments, the second medical device 20 transmits the medical data to the EMR computer or server 44 for population in the EMR 40. The controller 16 may also transmit the patient identification to the EMR computer or server 44 before the medical data is transmitted thereto by the second medical device 20 or simultaneously with the medical data so that the medical data may be properly populated in the EMR 40.

In this way, the controller 16 and/or the medical devices 18, 20 transmit the medical data to the electronic medical record 40 for storage therein. The controller 16 associates the medical devices 18, 20 with the patient 24 without manual association by the caregiver 22.

The first medical device 18 may comprise an infusion pump, a patient monitor, an ECG machine, a ventilator, a defibrillator, a vital sign monitoring system, a blood pressure monitor, a dialysis machine, a nebulizer, a pulse oximeter, a cardiac monitor, an ultrasound machine, a multipara monitor, a capnography monitor, a fetal monitor, a hospital bed, a bed, a stretcher, a wheelchair, a patient lift, an oxygen supply, or any other suitable medical device.

The second medical device 20 may comprise an infusion pump, a patient monitor, an ECG machine, a ventilator, a defibrillator, a vital sign monitoring system, a blood pressure monitor, a dialysis machine, a nebulizer, a pulse oximeter, a cardiac monitor, an ultrasound machine, a multipara monitor, a capnography monitor, a fetal monitor, a hospital bed, a bed, a stretcher, a wheelchair, a patient lift, an oxygen supply, or any other suitable medical device.

The controller 16 is configured to compare the characteristics of the medical devices 18, 20 with the patient characteristics. For example, upon detection of the identifiers 26, 28, the controller 16 receives characteristics of the medical devices 18, 20 from the hospital database 42. The characteristics of the medical devices 18, 20 may include the specific type of medical device 18, 20, the specific model of the medical device 18, 20, therapies provided by the medical device 18, 20, drugs administered by the medical device 18, 20, etc. The controller 16 may also receive the patient characteristics from the hospital database 42.

The controller 16 is configured to compare the characteristics of the medical devices 18, 20 with the patient characteristics to determine if the characteristics of the medical devices 18, 20 align with the patient characteristics. The controller 16 is configured to issue an alert in response to the characteristics of either of the medical devices 18, 20 not aligning with the patient characteristics. In this way, the controller 16 can issue the alert if one or more of the medical devices 18, 20 is not proper for the patient 24. If the alert is issued, the caregiver 22 may then intervene and provide the appropriate medical devices 18, 20.

For example, the patient characteristics may include the body mass index or weight of the patient 24, and the characteristics of the medical devices 18, 20 may include the specific type and model of a hospital bed. If the hospital bed does not have a weight capacity that aligns with the patient weight or body mass index of the patient 24, the controller 16 may issue the alert. As another example, the patient characteristics may include pressure sores, and the characteristics of the medical devices 18, 20 may include the specific type and model of a hospital bed. If the hospital bed does not help to alleviate pressure sores, the controller 16 may issue the alert.

The alert may comprise a visual notification on a computer, a visual notification on a mobile device, a visual notification on a tablet, a sound from the computer, a sound from the mobile device, a sound from the tablet, a sound from one or more of the medical device 18, 20, a sound from an intercom, a vibration from the mobile device, a vibration from the tablet, any other suitable alert, or any suitable combination of alerts.

As shown in FIG. 3, the controller 16 receives inputs related to the number of the room 32, the patient 24, the patient characteristics, the identifiers 26, 28, 30, and the medical device 18, 20 characteristics. The microprocessor 36 utilizes instructions stored in the memory 38 to associate the patient 24, the caregiver 22, and the medical devices 18, 20. The inputs to the controller 16 are from the hospital database 42 and the imaging device 14.

The controller 16 is also configured to disassociate the medical devices 18, 20, the caregiver 22, and the patient 24 from one another. For example, in response to the imaging device 14 no longer sensing, identifying, or detecting the first identifier 26, the controller 16 disassociates the first medical device 18 from the patient 24. This may occur when the first medical device 18 is removed from the room 32. In this way, the medical data from the first medical device 18 is no longer transmitted to the EMR computer or server 44 for population in the electronic medical record 40 of the patient 24.

As another example, in response to the imaging device 14 no longer sensing, identifying, or detecting the second identifier 28, the controller 16 disassociates the second medical device 20 from the patient 24. In response to the imaging device 14 no longer sensing, identifying, or detecting the third identifier 30, the controller 16 disassociates the caregiver 22 from the patient 24.

As shown in FIG. 2, the medical devices 18, 20 are also in communication with the hospital database 42. In this way, the medical devices 18, 20 also transmit the medical data to the hospital database 42.

It will be understood that the facility may comprise any number of rooms in addition to the room 32. Each room 32 in the facility may include at least one imaging device 14 therein. In this way, the medical devices 18, 20 and the caregiver 22 may be tracked as the medical devices 18, 20 and the caregiver 22 move between rooms 32. The first medical device 18 may be associated with the patient 24 in the room 32. The first medical device 18 may be removed from the room 32 and moved to a different room. The first medical device 18 may then be disassociated from the patient 24 in the room 32 and associated with the patient in the different room.

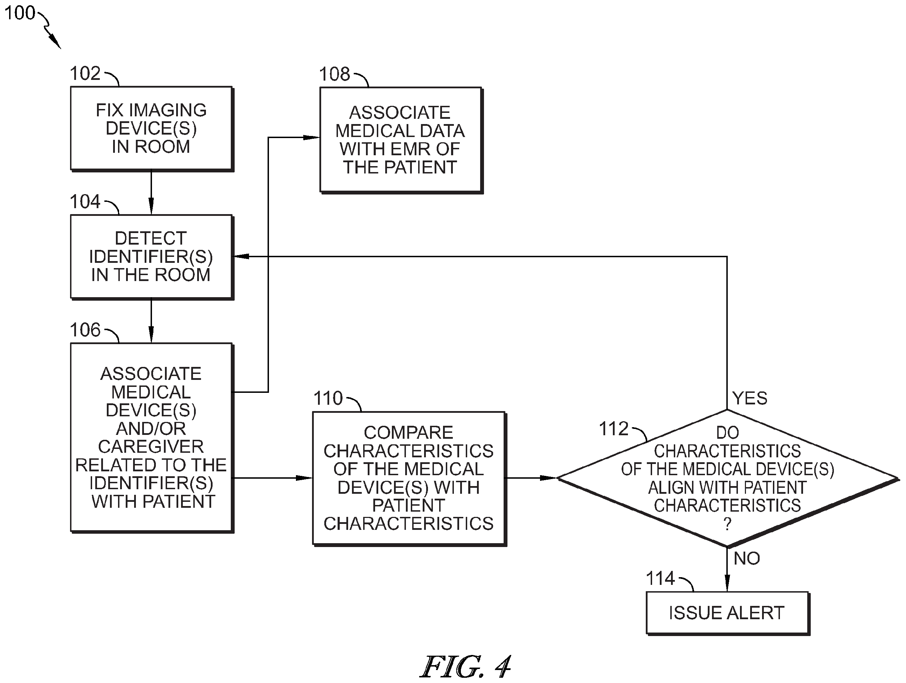

A method 100 is provided, as shown in FIG. 4. The method 100 includes step 102 of fixing the imaging device 14 within the room 32. The method 100 includes step 104 of detecting the plurality of identifiers 12 in the room 32 via the imaging device 14. Step 104 includes detecting the first identifier 26 associated with the first medical device 18 by the imaging device 14 upon entry into the room 32. Step 104 also includes detecting the second identifier 28 associated with the second medical device 20 by the imaging device 14 upon entry into the room 32. Step 104 also includes detecting the third identifier 30 associated with the caregiver 22 by the imaging device 14 upon entry into the room 32. Step 104 may include determining, via the controller 16, that the first medical device 18 is about to be used for and/or is being used for the patient 24 located within the room 32.

The method 100 includes step 106 of associating, via the controller 16, the medical devices 18, 20 and the caregiver 22 related to the detected identifiers 26, 28, 30 with the patient 24. Step 106 may include associating, via the controller 16, the first medical device 18 and the patient 24 with one another, associating, via the controller 16, the second medical device 20 and the patient 24 with one another, and/or associating, via the controller 16, the caregiver 22 and the patient 24 with one another.

The method 100 includes step 108 of automatically populating medical data from the medical devices 18, 20 in the electronic medical record 40 of the patient 24 via the controller 16 and/or the medical devices 18, 20. Step 108 may include transmitting, via the controller 16 and/or the medical devices 18, 20, the medical data from the medical devices 18, 20 and/or the controller 16 to the electronic medical record 40.

The method 100 includes step 110 of comparing, via the controller 16, characteristics of the medical devices 18, 20 with patient characteristics. The method 100 includes decision step 112 of determining, via the controller 16, if the characteristics of the medical devices 18, 20 align with the patient characteristics. If the characteristics of the medical devices 18, 20 do not align with the patient characteristics ('NO'), an alert is issued by the controller 16 in step 114. If the characteristics of the medical devices 18, 20 do align with the patient characteristics ('YES'), the method 100 proceeds to step 104 to continue to detect the plurality of identifiers 12 in the room 32 via the imaging device 14. In this way, the method 100 continuously detects the plurality of identifiers 12 and associates or disassociates, via the controller 16, the medical devices 18,20 and the caregiver 22 with the patient 24 depending on the detection of the plurality of identifiers 12.

The controller 16 may be embodied as any type of device or collection of devices capable of performing various computing functions. In some embodiments, the controller 16 may be embodied as a single device such as an integrated circuit, an embedded system, a field-programmable gate array (FPGA), a system-on-a-chip (SOC), or other integrated system or device. Additionally, in the illustrative embodiment, the controller 16 includes or is embodied as the processor 36 and the memory 38.

The processor 36 may be embodied as any type of processor capable of performing the functions described herein. For example, the processor 36 may be embodied as a single or multi-core processor(s), a microcontroller, or other processor or processing/controlling circuit. In some embodiments, the processor 36 may be embodied as, include, or be coupled to an FPGA, an application specific integrated circuit (ASIC), reconfigurable hardware or hardware circuitry, or other specialized hardware to facilitate performance of the functions described herein.

The memory 38 may be embodied as any type of volatile (e.g., dynamic random access memory (DRAM), etc.) or non-volatile memory or data storage capable of performing the functions described herein. Volatile memory may be a storage medium that requires power to maintain the state of data stored by the medium. In some embodiments, all or a portion of the memory 38 may be integrated into the processor 36. In operation, the memory 38 may store various software and data used during operation.

The controller 16 is communicatively coupled to other components of the medical data association system 10 via, for example, communication circuitry that enables communications over a network. The communication circuitry may be configured to use any one or more communication technology (e.g., wired or wireless communications) and associated protocols (e.g., Ethernet, InfiniBand^{®}, Bluetooth^{®}, Wi-Fi^{®}, WiMAX, 3G, 4G LTE, 5G, etc.) to effect such communication.

In the illustrative embodiment, the controller 16, the hospital database 42, the imaging device 14, the medical devices 18, 20 and the EMR computer 44 are in communication over a network, which may be embodied as any type of wired or wireless communication network, including local area networks (LANs) or wide area networks (WANs), digital subscriber line (DSL) networks, cable networks (e.g., coaxial networks, fiber networks, etc.), cellular networks (e.g., Global System for Mobile Communications (GSM), Long Term Evolution (LTE), Worldwide Interoperability for Microwave Access (WiMAX), 3G, 4G, 5G, etc.), radio area networks (RAN), global networks (e.g., the internet), or any combination thereof, including gateways between various networks.

When terms of degree such as "generally," "substantially," and "about" are used herein in connection with a numerical value or a qualitative term susceptible to a numerical measurement, it is contemplated that an amount that is plus or minus 10 percent, and possibly up to plus or minus 20 percent, of the numerical value, is covered by such language, unless specifically noted otherwise, to at least account for manufacturing tolerances. Otherwise, a suitable definition for the terms "generally," "substantially," and "about," is largely, but not necessarily wholly, the term specified.

When the terms "a" or "an" or the phrases "one or more" or "at least one" are used herein, including in the claims, they are all intended to be synonymous and mean that one, or more than one, of the thing recited may be present. Similarly, when the phrases "a plurality" or "two or more" or "at least two" or "a pair" are used, they are all intended to be synonymous and mean that two, or more than two, of the thing recited may be present. Furthermore, when the language "at least a portion" and/or "a portion" is used herein, the item can include a portion and/or the entire item unless specifically stated to the contrary.

According to this disclosure, phrases of the form "at least one of A and B" and "at least one of the following: A and B" and similar such phrases, mean "A alone, or B alone, or both A and B." Phrases of the form "at least one of A or B" and "at least one of the following: A or B" and similar such phrases, also mean "A alone, or B alone, or both A and B." Furthermore, phrases of the form "A and/or B" also mean "A alone, or B alone, or both A and B."

Embodiments of the invention can be described with reference to the following numbered clauses, with preferred features laid out in the dependent clauses:
Clause 1. A medical data association system comprising a plurality of identifiers including a first identifier associated with a first medical device and a second identifier different than the first identifier associated with a second medical device; an imaging device located in a room and configured to detect the plurality of identifiers located within the room, wherein the first identifier and the second identifier are visible to the imaging device and substantially imperceptible to unaided human observation; and a controller in communication with the imaging device and the first medical device, the controller configured to, upon determining that the first medical device is about to be used for a patient and/or is being used for the patient in the room, associate the first medical device and the patient with one another so that medical data from the first medical device can be populated in an electronic medical record (EMR) of the patient when transmitted to an EMR computer.
Clause 2. The medical data association system of clause 1, wherein the controller is configured to determine that the first medical device is about to be used and/or is being used based on detection of the first identifier within the room.
Clause 3. The medical data association system of clause 1, wherein the controller is configured to associate the first medical device and the patient with one another without manual association by a caregiver.
Clause 4. The medical data association system of clause 1, wherein, upon determining that the first medical device is about to be used and/or is being used, the first medical device is configured to transmit the medical data to the electronic medical record.
Clause 5. The medical data association system of clause 1, wherein the controller is configured to associate the second medical device with the patient upon detection of the second identifier within the room.
Clause 6. The medical data association system of clause 5, wherein the controller is configured to associate the second medical device and the patient with one another without manual association by a caregiver.
Clause 7. The medical data association system of clause 5, wherein the controller is configured to compare characteristics of the second medical device with patient characteristics.
Clause 8. The medical data association system of clause 7, wherein the controller is configured to issue an alert in response to the characteristics of the second medical device not aligning with the patient characteristics.
Clause 9. The medical data association system of clause 8, wherein the second medical device is a bed.
Clause 10. The medical data association system of clause 1, wherein the controller is configured to disassociate the first medical device and the patient in response to a lack of detection of the first identifier in the room.
Clause 11. The medical data association system of clause 10, wherein the controller is configured to disassociate the second medical device and the patient in response to a lack of detection of the second identifier in the room.
Clause 12. The medical data association system of clause 1, wherein the first identifier and the second identifier are formed of ultraviolet-reactive ink, and wherein the first identifier is applied to the first medical device and the second identifier is applied to the second medical device.
Clause 13. The medical data association system of clause 12, wherein the imaging device includes an ultraviolet camera.
Clause 14. The medical data association system of clause 1, wherein the controller includes a processor and a memory having instructions stored therein which, when executed by the processor, cause the processor to associate the first medical device and the patient with one another.
Clause 15. The medical data association system of clause 1, wherein the controller is configured to receive a first signal from the imaging device indicative of detection of the first identifier within the room.
Clause 16. The medical data association system of clause 15, wherein the controller is configured to receive a second signal from the imaging device indicative of detection of the second identifier within the room.
Clause 17. The medical data association system of clause 16, wherein, in response to the first signal, the controller is configured to associate the first medical device and the patient located within the room with one another, and in response to the second signal, the controller is configured to associate the second medical device and the patient located within the room with one another.
Clause 18. The medical data association system of clause 17, wherein, in response to the second signal, the controller is configured to compare characteristics of the second medical device with patient characteristics.
Clause 19. The medical data association system of clause 1, wherein the plurality of identifiers further includes a third identifier associated with a caregiver, and wherein the controller is configured to associate the caregiver and the patient with one another in response to detection of the third identifier by the imaging device.
Clause 20. The medical data association system of clause 19, wherein the controller is configured to determine that the first medical device is about to be used and/or is being used based on detection of the first identifier and the third identifier within the room at the same time.
Clause 21. The medical data association system of clause 19, wherein the first and second identifiers are formed of ultraviolet-reactive ink, and wherein the third identifier includes facial features of the caregiver.
Clause 22. The medical data association system of clause 19, wherein the first and second identifiers are formed of ultraviolet-reactive ink, and wherein the third identifier includes an identification badge of the caregiver.
Clause 23. The medical data association system of clause 19, wherein the first identifier, the second identifier, and the third identifier are formed of ultraviolet-reactive ink.
Clause 24. A method comprising positioning an imaging device within a room; detecting a first identifier associated with a first medical device by the imaging device upon entry of the first medical device into the room; detecting a second identifier associated with a second medical device by the imaging device upon entry of the second medical device into the room, wherein the first identifier and the second identifier are visible to the imaging device and substantially imperceptible to unaided human observation; determining, with a controller in communication with the imaging device, that the first medical device is about to be used for and/or is being used for a patient located within the room; in response to determining that the first medical device is about to be used and/or is being used, associating, with the controller, the first medical device and the patient with one another; and automatically populating medical data from the first medical device into an electronic medical record (EMR) of the patient.
Clause 25. The method of clause 24, wherein determining that the first medical device is about to be used and/or is being used includes detecting the first identifier within the room.
Clause 26. The method of clause 24, further comprising, after associating the first medical device and the patient with one another, transmitting the medical data from the first medical device to the electronic medical record.
Clause 27. The method of clause 24, further comprising, in response to detecting the second identifier, associating, with the controller, the second medical device and the patient with one another.
Clause 28. The method of clause 27, further comprising comparing, with the controller, characteristics of the second medical device with patient characteristics.
Clause 29. The method of clause 28, further comprising determining, with the controller, that the characteristics of the second medical device do not align with the patient characteristics.
Clause 30. The method of clause 29, further comprising issuing an alert, with the controller, in response to determining that the characteristics of the second medical device do not align with the patient characteristics.
Clause 31. The method of clause 30, wherein the second medical device is a bed.
Clause 32. The method of clause 24, further comprising disassociating, with the controller, the first medical device and the patient in response to a lack of detection of the first identifier in the room.
Clause 33. The method of clause 32, further comprising disassociating, with the controller, the second medical device and the patient in response to a lack of detection of the second identifier in the room.
Clause 34. The method of clause 24, further comprising applying the first identifier to the first medical device and applying the second identifier to the second medical device via ultraviolet-reactive ink.
Clause 35. The method of clause 34, wherein the imaging device includes an ultraviolet camera.
Clause 36. The method of clause 24, further comprising detecting a third identifier associated with a caregiver by the imaging device upon entry of the caregiver into the room.
Clause 37. The method of clause 36, further comprising, in response to detecting the third identifier, associating, with the controller, the caregiver and the patient with one another.
Clause 38. The method of clause 37, wherein determining that the first medical device is about to be used and/or is being used includes detecting the first identifier and the third identifier within the room at the same time.
Clause 39. The method of clause 37, wherein the first and second identifiers are formed of ultraviolet-reactive ink, and wherein the third identifier includes facial features of the caregiver.
Clause 40. The method of clause 37, wherein the first and second identifiers are formed of ultraviolet-reactive ink, and wherein the third identifier includes an identification badge of the caregiver.
Clause 41. The method of clause 37, wherein the first identifier, the second identifier, and the third identifier are formed of ultraviolet-reactive ink.
Clause 42. A controller comprising a processor, and a memory having instructions stored therein executable by the processor to cause the processor to (a) determine that a first identifier associated with a first medical device is located within a room, (b) determine that a second identifier associated with a second medical device is located within the room, (c) determine that the first medical device is about to be used for and/or is being used for a patient within the room, and (d) in response to determining that the first medical device is about to be used for and/or is being used for the patient within the room, associate the first medical device and the patient with one another so that medical data from the first medical device is automatically populated in an electronic medical record of the patient.
Clause 43. The controller of clause 42, wherein the instructions stored in the memory further include instructions executable by the processor to cause the processor to output a signal to the first medical device to cause the first medical device to transmit the medical data to the electronic medical record.
Clause 44. The controller of clause 42, wherein the instructions stored in the memory further include instructions executable by the processor to cause the processor to associate the second medical device and the patient with one another upon determining that the second identifier is located within the room.
Clause 45. The controller of clause 44, wherein the instructions stored in the memory further include instructions executable by the processor to cause the processor to compare characteristics of the second medical device with patient characteristics.
Clause 46. The controller of clause 45, wherein the instructions stored in the memory further include instructions executable by the processor to cause the processor to issue an alert in response to the characteristics of the second medical device not aligning with the patient characteristics.
Clause 47. The controller of clause 42, wherein the instructions stored in the memory further include instructions executable by the processor to cause the processor to disassociate the first medical device and the patient in response to a lack of detection of the first identifier in the room.
Clause 48. The controller of clause 47, wherein the instructions stored in the memory further include instructions executable by the processor to cause the processor to disassociate the second medical device and the patient in response to a lack of detection of the second identifier in the room.
Clause 49. The controller of clause 42, wherein the instructions stored in the memory further include instructions executable by the processor to cause the processor to determine that a third identifier associated with a caregiver is located within the room.
Clause 50. The controller of clause 49, wherein the instructions stored in the memory further include instructions executable by the processor to cause the processor to associate the caregiver and the patient with one another upon determination that the third identifier is located within the room.

## Claims

1. A medical data association system comprising
a plurality of identifiers including a first identifier associated with a first medical device and a second identifier different than the first identifier associated with a second medical device,
an imaging device located in a room and configured to detect the plurality of identifiers located within the room, wherein the first identifier and the second identifier are visible to the imaging device and substantially imperceptible to unaided human observation, and
a controller in communication with the imaging device and the first medical device, the controller configured to, upon determining that the first medical device is about to be used for a patient and/or is being used for the patient in the room, associate the first medical device and the patient with one another so that medical data from the first medical device can be populated in an electronic medical record (EMR) of the patient when transmitted to an EMR computer.

2. The medical data association system of claim 1, wherein the controller is configured to determine that the first medical device is about to be used and/or is being used based on detection of the first identifier within the room.

3. The medical data association system of either claim 1 or claim 2, wherein the controller is configured to associate the first medical device and the patient with one another without manual association by a caregiver.

4. The medical data association system of any proceeding claim, wherein the controller is configured to associate the second medical device with the patient upon detection of the second identifier within the room.

5. The medical data association system of claim 4, wherein the controller is configured to compare characteristics of the second medical device with patient characteristics, and wherein the controller is configured to issue an alert in response to the characteristics of the second medical device not aligning with the patient characteristics.

6. The medical data association system of claim 5, wherein the second medical device is a bed.

7. The medical data association system of any proceeding claim, wherein the controller is configured to disassociate the first medical device and the patient in response to a lack of detection of the first identifier in the room.

8. The medical data association system of claim 7, wherein the controller is configured to disassociate the second medical device and the patient in response to a lack of detection of the second identifier in the room.

9. The medical data association system of any proceeding claim, wherein the first identifier and the second identifier are formed of ultraviolet-reactive ink, and wherein the first identifier is applied to the first medical device and the second identifier is applied to the second medical device.

10. The medical data association system of claim 9, wherein the imaging device includes an ultraviolet camera.

11. The medical data association system of any proceeding claim, wherein the plurality of identifiers further includes a third identifier associated with a caregiver, and wherein the controller is configured to associate the caregiver and the patient with one another in response to detection of the third identifier by the imaging device.

12. The medical data association system of claim 11, wherein the controller is configured to determine that the first medical device is about to be used and/or is being used based on detection of the first identifier and the third identifier within the room at the same time.

13. The medical data association system of either claim 11 or claim 12, wherein the first and second identifiers are formed of ultraviolet-reactive ink, and wherein the third identifier includes facial features of the caregiver.

14. The medical data association system of either claim 11 or claim 12, wherein the first and second identifiers are formed of ultraviolet-reactive ink, and wherein the third identifier includes an identification badge of the caregiver.

15. The medical data association system of either claim 11 or claim 12, wherein the first identifier, the second identifier, and the third identifier are formed of ultraviolet-reactive ink.
